(19) Europäisches Patentamt
European Patent Office
Office européen des brevets

(11) Publication number: **0 197 282 A1**

(12) **EUROPEAN PATENT APPLICATION**

(21) Application number: 86102535.1

(22) Date of filing: 27.02.86

(51) Int. Cl.[4]: C 07 D 487/04
C 07 D 487/14, C 07 D 271/06
//A61K31/55, (C07D487/04, 243:00, 235:00), (C07D487/14, 243:00, 235:00, 209:00), (C07D487/14, 243:00, 235:00, 205:00), (C07D487/14, 243:00, 235:00, 221:00)

(30) Priority: 08.03.85 DK 1080/85
08.03.85 DK 1081/85
17.05.85 DK 2203/85
17.05.85 DK 2204/85
17.10.85 DK 4769/85

(43) Date of publication of application:
15.10.86 Bulletin 86/42

(84) Designated Contracting States:
AT BE CH DE FR GB IT LI LU NL SE

(71) Applicant: A/S FERROSAN
Sydmarken 5
DK-2860 Soeborg(DK)

(72) Inventor: Watjen, Frank
Josteinsvej 27
DK-2860 Bagsvaerd(DK)

(72) Inventor: Engelstoft, Mogens
Mosegard Park 121
DK-3500 Vaelose(DK)

(72) Inventor: Hansen, Bondo Hansen
Havretoften 10
DK-2800 Lyngby(DK)

(72) Inventor: Jensen, Helth Leif
Bogehoj 37
DK-2900 Hellerup(DK)

(74) Representative: Patentanwälte Grünecker, Kinkeldey, Stockmair & Partner
Maximilianstrasse 58
D-8000 München 22(DE)

(54) **Novel oxadiazolyl imidazobenzodiazepine derivatives and methods of preparing such compounds.**

(57) Novel oxadiazolyl imidazobenzsodiazepine derivatives having the formula

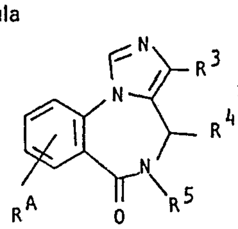

wherein $R^3$ has the formula

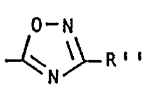

or

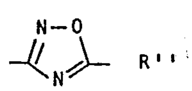

wherein R" is hydrogen, $C_{1-6}$-alkyl, $C_{1-6}$-alkoxymethyl or $C_{3-6}$-cycloalkyl;
$R^4$ is hydrogen;
$R^5$ is $C_{1-6}$-alkyl or $R^4$ and $R^5$ together form a 2-4 membered alkylene chain; and
$R^A$ is $_{1-6}$-alkyl, $C_{1-6}$-alkoxy or $C_{1-3}$-trifluoroalkyl.

The compounds are suitable for use in psychopharmaceutical preparations as anticonvulsants, anxiolytics, hypnotics and nootropics.

EP 0 197 282 A1

Novel oxadiazolyl imidazobenzodiazepine derivatives and methods of preparing such compounds.

This invention relates to novel oxadiazolyl imidazobenzodiazepine derivatives and to methods of preparing such compounds. These novel compounds are suitable for use in psychopharmaceutical preparations as anticonvulsants, anxiolytics, hypnotics and nootropics.

The novel compounds of the invention are oxadiazolyl imidazobenzodiazepine derivatives having the general formula I:

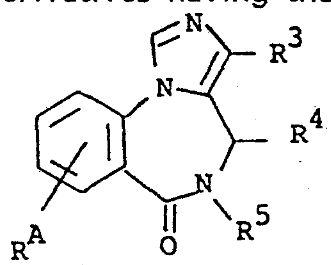

(I)

wherein $R^3$ has the formula

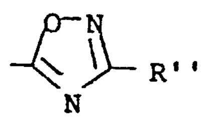

or

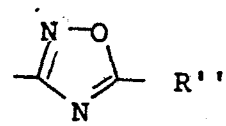

wherein R'' is hydrogen, $C_{1-6}$-alkyl, $C_{1-6}$-alkoxymethyl or $C_{3-6}$-cycloalkyl;
$R^4$ is hydrogen;
$R^5$ is $C_{1-6}$-alkyl or wherein $R^4$ and $R^5$ together form a 2-4 membered alkylene chain; and wherein $R^A$ is $C_{1-6}$-alkyl, $C_{1-6}$-alkoxy or $C_{1-3}$--trifluoroalkyl.

European patent application No. 109.921 discloses various oxadiazolyl derivatives of imidazobenzodiazepines, which are capable of displacing flunitrazepam from benzodiazepine receptors.

Also European patent application No. 150.040 discloses oxadiazolyl derivatives of imidazobenzodiazepines. Although the generic claims of this patent application include compounds having the general formula II

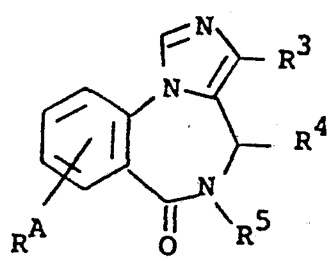

(II)

wherein $R^3$, $R^4$, $R^5$ and $R^A$ have the meanings defined above. European patent application No. 150.040 contains no specific disclosure of compounds, wherein $R^A$ is alkoxy or lower alkyl.

It has been found that the novel compounds of the invention have improved pharmaceutical properties compared to well known related compounds.

It is well known (Squires, R.F. and Braestrup, C., Nature (London) 266, (1977) 734) that specific sites in the central nervous systems of vertebrates exhibit a high specific affinity for binding 1,4- and 1,5-benzodiazepines. These sites are called benzodiazepine receptors.

The pharmaceutical potency of the compounds of the invention can be illustrated by determining the capability for displacing radioactively labelled flunitrazepam and the imidazobenzodiazepine $^{3}H$-RO 15-1788 from such benzodiazepine receptors.

The displacement activity of the compounds of the invention has been determined by determining the $IC_{50}$ and $ED_{50}$ values. The $IC_{50}$ value represents the concentration (nM, 30°C) which causes a displacement of 50% of the specific binding of $^{3}H$-RO 15-1788 from benzodiazepine receptors in samples comprising a total volume of 1 ml.

The displacement test is performed as follows:

750 µl of rat cerebral cortical membrane homogenate was incubated with 100 µl of 5 nM $^{3}H$-RO 15-1788 in water at 30°C. 100 µl of a solution of the test compound and 50 µl of Krebs buffer were added. After incubation the binding reaction was terminated by filtration through Whatman GF/B glass fibre filters followed by 2x5 ml wash with ice-cold buffer and the radioactivity was measured by scintillation counting. The $IC_{50}$ was determined on the basis of at least 4 concentrations of the test compound and by log/probit analysis of the resulting data.

The $ED_{50}$ value represents the dose (mg/kg) of a test substance which causes the specific binding of flunitrazepam to benzodiazepine receptors in a living brain to be reduced to 50% of the control value.

Such an in vivo test is carried out as follows:

Groups of mice were injected with the test substance at different doses and usually subcutaneously. 15 Minutes later $^{3}H$-flunitrazepam was given intravenously to the mice and after further 20 minutes the mice were killed, their forebrain membranes were removed and the radioactivity of these forebrain membranes was measured by scintillation counting. The $ED_{50}$ value was determined from dose-

response curves.

The results obtained by subjecting some of the compounds of the invention to the above mentioned tests will appear from the following table 1.

Table 1

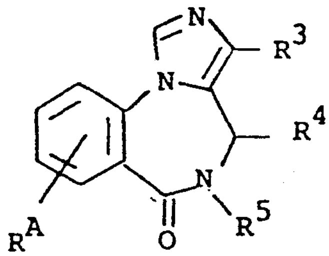

(I)

| 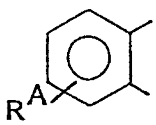 | $R^4$ | $R^5$ | $R^3$ | in vitro $IC_{50}$ nM | in vivo $ED_{50}$ mg/kg |
|---|---|---|---|---|---|
| 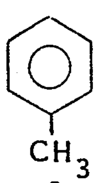 | H | $CH_3$ | 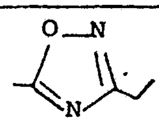 | 44.8 | 2.2 |
| 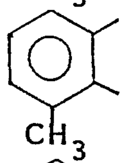 | $-CH_2CH_2-$ | | 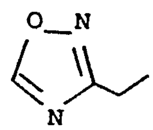 | 30 | 0.4 |
| 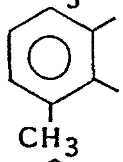 | $-CH_2CH_2-$ | | 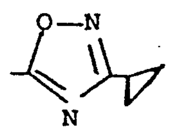 | 0.2 | 0.7 |
| 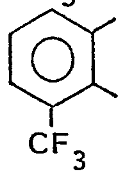 | H | $CH_3$ | 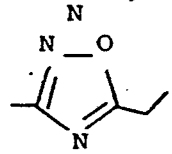 | 5.6 | 0.5 |

The invention also relates to methods of preparing the above mentioned compounds. These methods comprise

a) reacting a reactive derivative of a compound having the general formula III

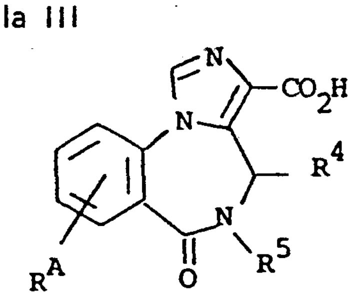

(III)

wherein $R^4$, $R^5$ and $R^A$ have the meanings set forth above with a compound having the formula IV

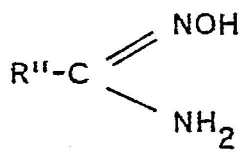

(IV)

wherein R'' has the meaning set forth above to form a compound having the formula I in which $R^3$ is

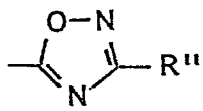

wherein R'' has the meaning set forth above,

b) reacting a compound having the general formula V

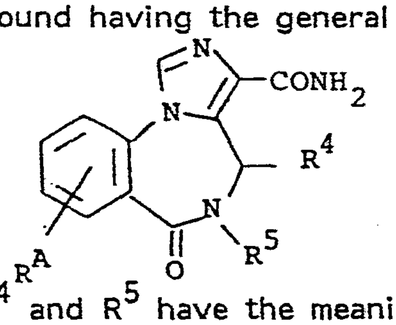

(V)

wherein $R^A$, $R^4$ and $R^5$ have the meanings set forth above with a compound of the formula VI

$$R''\text{-}C(OCH_3)_2N(CH_3)_2 \qquad (VI)$$

wherein R'' has the meaning set forth above, to form a compound having the general formula VII

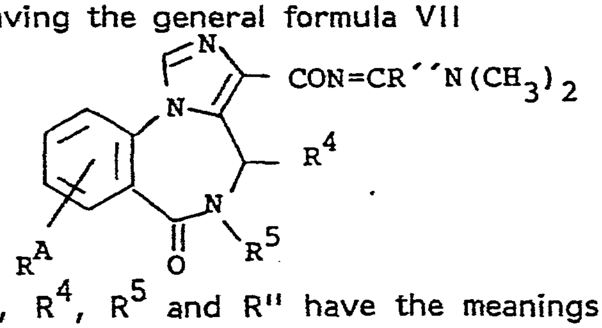

(VII)

wherein $R^A$, $R^4$, $R^5$ and R'' have the meanings set forth above and reacting the compound having the formula VII with $NH_2OH$ or another aminating agent, to form a compound having the formula I in which $R^3$ is

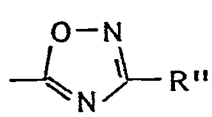

wherein R'' has the meaning set forth above,

c) reacting a compound having the general formula VIII

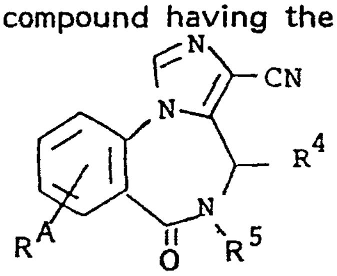

(VIII)

wherein $R^A$, $R^4$ and $R^5$ have the meanings set forth above with $NH_2OH$, to form a compound having the general formula IX

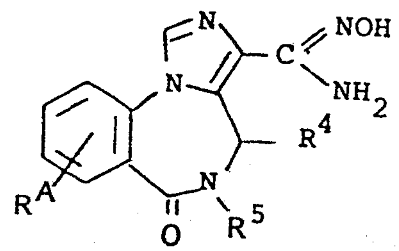

(IX)

wherein $R^A$, $R^4$ and $R^5$ have the meanings set forth above, and reacting the compound having the formula IX with a compound having the general formula X

$$(R''CO_2)O \qquad (X)$$

wherein R'' has the meaning set forth above, to form a compound having the formula I in which $R^3$ is

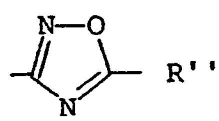

wherein R'' has the meaning set forth above or

d) reacting a compound having the general formula XI

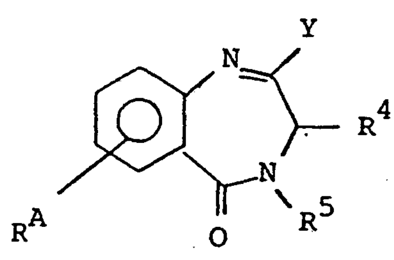

(XI)

wherein $R^4$, $R^5$ and $R^A$ have the meanings defined above, and Y is a leaving group with a compound having the formula XII

$$CN - CH_2 - R^3 \qquad (XII)$$

wherein $R^3$ has the meaning defined above, to form a compound having the formula I.

The starting materials for use in the methods of the invention can be prepared from commercially available benzene derivatives by methods described in European patent applications Nos. 109.921 and 27.214 and Synthesis, Vol. 10, pp 681-2.

The compounds of this invention can be used for the formulation of pharmaceutical preparations, e.g. for oral and parenteral administration to mammals including humans, in accordance with conventional methods of galenic pharmacy.

Conventional exipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium steárate, talc, silicic acid, fatty acid monoglycerides and diglycerides, pentaerythritol fatty acid esters, hydroxymethylcellulose and polyvinylpyrrylidone.

The pharmaceutical preparations can be sterilized and mixed, if desired, with auxiliary agents, such as lubricants, preservatives, stabilizers, wetting agents, emulsifiers, salts for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are conveniently unit dosages.

For oral application, particularly suitable are tablets, dragees or capsules comprising a talc and/or a carbohydrate carrier or binder or the like, the carrier preferably being lactose and/or corn starch and/or potato starch. A syrup, elixir or the like can be used in cases where a sweetened vehicle can be employed.

Generally, the compounds of this invention are dispensed in unit dosage form comprising 0.05-100 mg in a pharmaceutically acceptable carrier per unit dosage.

The dosage of the compounds according to this invention is 0.1-300 mg/day, preferably 1-30 mg/day, when administered to patients, e.g. humans, as a drug.

A representative tablet which may be prepared by conven-

tional tabletting techniques contains:

| | | |
|---|---|---|
| Active compound | 1.0 | mg |
| Lactosum | 67.8 | mg Ph.Eur. |
| Avicel ® | 31.4 | mg |
| Amberlite ® IRP 88 | 1.0 | mg |
| Magnesii stearas | 0.25 | mg Ph.Eur. |

The preparation of the compounds of the invention will now be described in further detail with reference to the following examples:

## Example 1

A. Isatoic anhydride

7.5 g of 2-aminobenzoic acid hydrochloride was mixed with 10 ml of diphosgen and the mixture was stirred in 150 ml dioxan for 40 minutes at reflux. The resulting mixture was cooled and filtered.

Yield: 5.7 g of title compound.

The following compounds were synthesized from the appropriate aminobenzoic acids in a similar manner:

6-methylisatoic anhydride,
6-methoxyisatoic anhydride, and
6-trifluormethylisatoic anhydride,

5-methylisatoic anhydride,
5-methoxyisatoic anhydride, and
5-trifluoromethylisatoic anhydride.

B. 3,4-dihydro-4-methyl-2H-1,4-benzodiazepine-2,5(1H)-dione

64.8 g of isatoic acid anhydride was mixed with 35.4 g of sarcosine and the resulting mixture stirred with 420 ml dimethylsulfoxide at 100$^{o}$C for 4 hours. The mixture was cooled and was poured into 1.5 l water. The precipitated product was washed with water and dried.

Yield: 57.1 g of title compound.

The following compounds were synthesized from appropriate isatoic anhydride derivatives in a similar manner:

6-methoxy-3,4-dihydro-4-methyl-2H-1,4-benzodiazepine-2,5(1H)-dione,

(S)-6-methyl-1,2,3,11a-tetrahydro-5H-pyrrolo(2,1-c)(1,4)benzodiazepine-5,11(10H)-dione by reaction with L-proline,
M.p. 207.6-209.9°C.

6-trifluormethyl-3,4-dihydro-4-methyl-2H-1,4-benzodiazepine-2,5(1H)-dione,
M.p. 223.7-225.9°C.

7-methyl-3,4-dihydro-4-methyl-2H-1,4-benzodiazepine-2,5(1H)-dione,
M.p. 260.0-260.6°C.

(S)-7-methyl-1,2,3,11a-tetrahydro-5H-pyrrolo(2,1-c)(1,4)benzodiazepine-5,11(10H)-dione by reaction with L-proline,
M.p. 243.1-244.5°C.

6-methyl-3,4-dihydro-4-methyl-2H-1,4-benzodiazepine-2,5(1H)-dione,
M.p. 204.4-205.4°C.

(S)-6-methyl-1,10a-dihydro-azeto(2,1-c)(1,4)benzodiazepine-4,10(2H,9H)-dione by reaction with L-azetidine.

7-methoxy-3,4-dihydro-4-methyl-2H-1,4-benzodiazepine-2,5(1H)-dione,
M.p. 206°C.

(S)-7-methoxy-1,2,3,11a-tetrahydro-5H-pyrrolo(2,1-c)(1,4)benzodiazepine-5,11(10H)-dione by reaction with L-proline,
M.p. 216.8-217.6°C.

(S)-5-methyl-1,10a-dihydro-azeto(2,1-c)(1,4)benzodiazepine-4,10-(2H,9H)-dione by reaction with L-azetidine.

7-trifluormethyl-3,4-dihydro-4-methyl-2H-1,4-benzodiazepine-2,5(1H)-dione.

9-methyl-3,4-dihydro-4-methyl-2H-1,4-benzodiazepine-2,5(1H)-dione.

C. Ethyl 5,6-dihydro-5-methyl-6-oxo-4H-imidazo(1,5-a)(1,4)benzodiazepine-3-carboxylate

16.5 g of 4-methyl-3,4-dihydro-2H-1,4-benzodiazepine-2,5(1H)-dione and 11.7 g of K-t-butoxide was dissolved in 100 ml of dry dimethyl formamide (DMF) and the mixture was stirred for 10 minutes. Subsequently, 13.2 ml of diethylchlorophosphate was added and the resulting mixture was cooled to -20°C and stirred for 10 minutes.

A mixture of 10.8 g K-t-butoxide and 10.5 ml ethyl isocyanoacetate in 30 ml of dry DMF was added to the above prepared mixture at -10 to -20°C and the resulting mixture was stirred for one hour at room temperature, whereafter it was poured into 8.7 ml acetic acid in 300 ml water. This mixture was extracted twice with 150 ml methylene chloride. The organic phase was dried and evaporated. The resulting residue was crystallized leaving 10 g of the title compound as crystals.

The following compounds were synthesized from the appropriate benzodiazepine-diones in a similar manner:

Ethyl 5,6-dihydro-5-methyl-6-oxo-7-methoxy-4H-imidazo(1,5-a)(1,4)-benzodiazepine-3-carboxylate.

Ethyl (S)-8-methyl-11,12,13,13a-tetrahydro-9-oxo-9H-imidazo(1,5-a)-pyrrolo(2,1-c)(1,4)benzodiazepine-1-carboxylate,
M.p. 150.4-150.5°C.

Ethyl 5,6-dihydro-5-methyl-6-oxo-7-methoxy-4H-imidazo(1,5-a)(1,4)-benzodiazepine-3-carboxylate, as an oil.

Ethyl 8-methyl-5,6-dihydro-5-methyl-6-oxo-4H-imidazo(1,5-a)(1,4)-benzodiazepine-3-carboxylate,
M.p. 195.5-195.8°C.

Ethyl (S)-7-methyl-11,12,13,13a-tetrahydro-9-oxo-9H-imidazo(1,5-a)-pyrrolo(2,1-c)(1,4)benzodiazepine-1-carboxylate,
M.p. 271.0-271.7°C.

Ethyl 7-methyl-5,6-dihydro-5-methyl-6-oxo-4H-imidazo(1,5-a)(1,4)-benzodiazepine-3-carboxylate,
M.p. 147.7-148.1°C.

Ethyl (S)-7-methyl-10,11,12,12a-tetrahydro-9-oxo-9H-imidazo(1,5-a)-azeto(2,1-c)(1,4)benzodiazepine-1-carboxylate,
M.p. 257.6-259.1$^{o}$C.

Ethyl 8-methoxy-5,6-dihydro-5-methyl-6-oxo-4H-imidazo(1,5-a)(1,4)-benzodiazepine-3-carboxylate,
M.p. 228.1$^{o}$C.

Ethyl (S)-7-methoxy-11,12,13,13a-tetrahydro-9-oxo-9H-imidazo(1,5-a)-pyrrolo(2,1-c)(1,4)benzodiazepine-1-carboxylate,
M.p. 196.6-197.1$^{o}$C.

Ethyl (S)-8-methyl-10,11,12,12a-tetrahydro-9-oxo-9H-imidazo(1,5-a)-azeto(2,1-c)(1,4)benzodiazepine-1-carboxylate,
M.p. 166.0$^{o}$C.

Ethyl 7-methoxy-5,6-dihydro-5-methyl-6-oxo-4H-imidazo(1,5-a)(1,4)-benzodiazepine-3-carboxylate, as an oil.

Ethyl 10-methyl-5,6-dihydro-5-methyl-6-oxo-4H-imidazo(1,5-a)(1,4)-benzodiazepine-3-carboxylate,
M.p. 196.3-196.9$^{o}$C.

D. Methoxyacetamide oxime

2.3 g of sodium in 33 ml of dry methanol and 6.65 g of hydroxylamine hydrochloride in 66 ml of dry methanol were mixed. 7.8 g of methoxyacetonitrile was added dropwise to the filtrate. The mixture was left for 48 hours. The mixture was then cooled to 4$^{o}$C. Filtration and evaporation of the filtrate gave 8.7 g of the title compound.

The following compounds were synthesized from the appropriate nitriles in a similar manner:

Propionamide oxime,
Iso-propyl carboxamide oxime,
Acetamide oxime,
Valeroamide oxime,
Cyclopropyl carboxamide oxime.

E. 3-(5-(3-methoxymethyl-1,2,4-oxadiazole)-yl)-5,6-dihydro-5-methyl-6-oxo-4H-imidazo(1,5-a)(1,4)benzodiazepine

240 mg of sodium was dissolved in 12 ml of dry ethanol together with 4 g of a molecular sieve (4Å). 2.2 g of methoxyacetamide oxime and 1 g of ethyl 5,6-dihydro-5-methyl-6-oxo-4H-imidazo(1,5-a)(1,4)-benzodiazepine-3-carboxylate in 5 ml of dry ethanol were added. The mixture was refluxed for 15 hours and was then evaporated. The residue was recrystallized from water yielding 0.6 g of the title compound.

M.p. 193.8-194.1°C.

The following compounds were synthesized from the appropriate carboxylates in a similar manner:

3-(5-(3-ethyl-1,2,4-oxadiazol)-yl)-8-trifluoromethyl-5,6-dihydro-5--methyl-6-oxo-4H-imidazo(1,5-a)(1,4)benzodiazepine.

M.p. 172-175°C.

3-(5-(3-ethyl-1,2,4-oxadiazole)-yl)-8-methyl-5,6-dihydro-5-methyl--6-oxo-4H-imidazo(1,5-a)(1,4)benzodiazepine.

M.p. 195.4-195.7°C.

(S)-1-(5-(3-ethyl-1,2,4-oxadiazole)-yl)-7-methyl-11,12,13,13a-tetrahydro-9-oxo-9H-imidazo(1,5-a)pyrrolo(2,1-c)(1,4)-benzodiazepine.

M.p. 270°C.

3-(5-(3-methoxymethyl-1,2,4-oxadiazole)-yl)-8-methyl-5,6-dihydro-5--methyl-6-oxo-4H-imidazo(1,5-a)(1,4)benzodiazepine.

M.p. 215.3-216.1°C.

(S)-1-(5-(3-ethyl-1,2,4-oxadiazole)-yl)-8-methyl-11,12,13,13a-tetrahydro-9-oxo-9H-imidazo(1,5-a)pyrrolo(2,1-c)(1,4)benzodiazepine.

M.p. 170.3-170.5°C.

3-(5-(3-ethyl-1,2,4-oxadiazole)-yl)-7-methyl-5,6-dihydro-5-methyl-6--oxo-4H-imidazo(1,5-a)(1,4)benzodiazepine.

M.p. 164.1°C.

(S)-1-(5-(3-ethyl-1,2,4-oxadiazole)-yl)-7-methyl-10,11,12,12a-tetrahydro-9-oxo-9H-imidazo(1,5-a)azeto(2,1-c)(1,4)benzodiazepine.

M.p. 210.1-212.4°C.

(S)-1-(5-(3-isopropyl-1,2,4-oxadiazole)-yl)-7-methyl-10,11,12,12a-tetrahydro-9-oxo-9H-imidazo(1,5-a)azeto(2,1-c)(1,4)benzodiazepine.

M.p. 193.4-195.4°C.

3-(5-(3-methyl-1,2,4-oxadiazole)-yl)-8 methoxy-5,6-dihydro-5-methyl--6-oxo-4H-imidazo(1,5-a)(1,4)benzodiazepine.

M.p. 222-222.3°C.

3-(5-(3-ethyl-1,2,4-oxadiazole)-yl)-8-methoxy-5,6-dihydro-5-methyl-6-oxo-4H-imidazo(1,5-a)(1,4)benzodiazepine.

M.p. 209.3-210.4°C.

(S)-1-(5-(3-ethyl-1,2,4-oxadiazole)-yl)-7-methoxy-11,12,13,13a-tetrahydro-9-oxo-9H-imidazo(1,5-a)pyrrolo(2,1-c)(1,4)benzodiazepine.

M.p. 237-238°C.

(S)-1-(5-(3-ethyl-1,2,4-oxadiazole)-yl)-8-methyl-10,11,12,12a-tetrahydro-9-oxo-9H-imidazo(1,5-a)azeto(2,1-c)(1,4)benzodiazepine.

M.p. 204.4-204.6°C.

3-(5-(3-ethyl-1,2,4-oxadiazole)-yl)-7-methoxy-5,6-dihydro-5-methyl-6-oxo-4H-imidazo(1,5-a)(1,4)benzodiazepine.

M.p. 211.3-213.0°C.

3-(5-(3-ethyl-1,2,4-oxadiazole)-yl)-10-methyl-5,6-dihydro-5-methyl-6--oxo-4H-imidazo(1,5-a)(1,4)benzodiazepine,

M.p. 162.6-163.3°C.

(S)-1-(3-cyclopropyl-1,2,4-oxadiazole-5-yl)-8-methyl-11,12,13,13a-tetrahydro-9-oxo-9H-imidazo(1,5-a)pyrrolo(2,1-c)(1,4)benzodiazepine.

M.p. 171.1-171.2°C.

3-(3-cyclopropyl-1,2,4-oxadiazole-5-yl)-5,6-dihydro-5-methyl-6-oxo-7--methoxy-4H-imidazo(1,5-a)(1,4)benzodiazepine.

M.p. 161.3°C.

3-(3-cyclopropyl-1,2,4-oxadiazole-5-yl)-5,6-dihydro-5-methyl-6-oxo-7--methyl-4H-imidazo(1,5-a)(1,4)benzodiazepine.

M.p. 152.5-153.1°C.

3-(3-isopropyl-1,2,4-oxadiazole-5-yl)-5,6-dihydro-5-methyl-6-oxo-7--methyl-4H-imidazo(1,5-a)(1,4)benzodiazepine.

M.p. 173.2-175.9°C.

(S)-1-(3-cyclopropyl-1,2,4-oxadiazole-5-yl)-8-methyl-10,11,12,12a-tetrahydro-9-oxo-9H-imidazo(1,5-a)azeto(2,1-c)(1,4)benzodiazepine.

M.p. 173.1-174.5°C.

## Example 2

### A. 3-Carbamoyl-8-methyl-5,6-dihydro-5-methyl-6-oxo-4H-imidazo-(1,5-a)(1,4)benzodiazepine

A mixture of 3.5 g imidazole and 0.95 ml thionylchloride was stirred for 15 minutes in 35 ml of tetrahydrofuran. The mixture was filtered and the filtrate was added to 1.7 g of 8-methyl-5,6-dihydro-5-methyl-6-oxo-4H-imidazo(1,5-a)(1,4)benzodiazepine-3-carboxylic acid in 4 ml DMF. This mixture was stirred for 2 hours at room temperature and $NH_3$ gas was supplied to the mixture for 15 minutes. The mixture thus obtained was then reduced to 15 ml and 100 ml of water was added. The precipitate was washed with water.

Yield. 1.6 g. M.p. 290-294°C.

### B. 3-Cyano-8-methyl-5,6-dihydro-5-methyl-6-oxo-4H-imidazo(1,5--a)(1,4)benzodiazepine

0.4 ml $Br_2$ in 10 ml methylene chloride was added to a solution of triphenyl phosphine in 40 ml methylene chloride at 0°C. The product of A was added to the mixture thus obtained together with 3.3 ml triethylamine. The mixture was stirred at room temperature for 30 minutes. Then 100 ml of water was added. The organic phase was reduced to 20 ml at reduced pressure and 1.2 g of the title compound precipitated by adding 50 ml of ether.

M.p. 252-252.3°C.

### C. 8-methyl-5,6-dihydro-5-methyl-6-oxo-4H-imidazo(1,5-a)(1,4)-benzodiazepine-3-carboxamide oxime

1,2 g of the product of B, 600 mg of hydroxyl amine

hydrochloride and 600 mg of potassium carbonate were stirred in 50 ml of 96% ethanol and 2 ml of water at $50^{o}C$ for 4 hours. Then further 300 ml of hydroxyl amine hydrochloride was added and the mixture was stirred for 1 hour. The mixture was then reduced at reduced pressure to 20 ml and 50 ml of water was added, whereupon 1.2 g of the title compound precipitated.

M.p. 227-229°C.

D. 3-(5-ethyl-1,2,4-oxadiazole-3-yl)-8-methyl-5,6-methyl-6-oxo-4H--imidazo(1,5-a)(1,4)benzodiazepine

A mixture of 450 mg of the product of C and 15 ml of propionic anhydride was stirred at 100°C for 10 minutes. Then 25 ml of dry ethanol, 3 g of a molecular sieve (3Å) and 50 mg of sodium was added and the resulting mixture was refluxed for 4 hours. The mixture was then filtered and the filtrate was reduced to 10 ml. Then 70 ml of water was added which caused the title compound to precipitate. The precipitate was washed with water and petroleum ether.

Yield 150 mg. M.p. 174.6-176.4°C.

The following compound was synthesized in a similar manner by reaction with acetic anhydride:

3-(3-(5-methyl-1,2,4-oxadiazole)-yl)-8-methyl-5,6-dihydro-5-methyl--6-oxo-4H-imidazo(1,5-a)(1,4)benzodiazepine.

M.p. 276°C.

## Example 3

A. 3-Cyclopropyl-5-isocyanomethyl-1,2,4-oxadiazole

a. 3-Cyclopropyl-5-formylaminomethyl-1,2,4-oxadiazole.

A solution of ethyl formylaminomethyl-carboxylate (150 mmol) and cyclopropyl carboxamide oxime (100 mmol) in 100% EtOH (100 ml) was charged with Na (200 mg) and a crushed molecular sieve (4Å) (10 g). The mixture thus obtained was stirred and heated to reflux for 8 hours. The mixture was cooled to room temperature, filtered through filter aid and the filtrate was evaporated in vacuo. The oily residue was partitionated into a $CHCl_3$ phase which was dried with $Na_2SO_4$ and evaporated.

b. 3-Cyclopropyl-5-isocyanomethyl-1,2,4-oxadiazole

A stirred solution of 3-cyclopropyl-5-formylaminomethyl-1,2,4-oxadiazole (60 mmol) and triethylamine (176 mmol) in $CH_2Cl_2$ (100 ml) was charged dropwise with $POCl_3$ (60 mmol) at $0^oC$. The mixture was then left for 30 minutes with stirring at $0^oC$, whereafter a solution of $Na_2CO_3$ (60 mmol) in $H_2O$ (50 ml) was added. The mixture was heated to room temperature, whereafter the organic phase was separated, dried and evaporated in vacuo. The residue was treated with ether, decanted and the solution was evaporated to give the title compound as an oil.

The oil was processed without any further purification.
IR: $cm^{-1}$: 2160.

3-ethyl-5-isocyanomethyl-1,2,4-oxadiazole was prepared from 3-ethyl-5-formylaminomethyl-1,2,4-oxadiazole in a similar manner.
IR: $cm^{-1}$: 2170.

B. 5-Cyclopropyl-3-isocyanomethyl-1,2,4-oxadiazole

a. Formylaminomethyl-carboxamide oxime

0.55 Mol of freshly liberated hydroxylamine dissolved in 370 ml methanol was added to 53.6 g (0.638 mol) N-formylamino-acetonitrile. An ice bath was used to keep the temperature below $20^oC$ during addition. The solution was allowed to stand at room temperature overnight, whereafter it was evaporated to give the title compound as pale crystals.
Decomp. 104-110$^oC$.

b. 3-Formylaminomethyl-5-ethyl-1,2,4-oxadiazole

A mixture of 70 ml ethyl propionate, 20 g formylamino-methylcarboxamide oxime, 1 g sodium and 30 g of a crushed molecular sieve (4Å) was refluxed in 300 ml abs. EtOH for 5 hours. The reaction mixture was filtered and the filtrate was evaporated. The oily residue was suspended in 300 ml $CHCl_3$, filtered and the filtrate was evaporated to give the title compound as an oil.

HNMR (60 MHz, $CDCl_3$) δ (ppm): 1.4 (3H, t, J=8 Hz), 2.9 (2H, q,J = Hz), 4.55 (2H, s), 7.8 (1H), broad-NH), 8.25

(1H, s).

The following compounds were synthesized from the appropriate ethyl esters in a similar manner:

3-Formylaminomethyl-5-cyclopropyl-1,2,4-oxadiazole. H-NMR (60 MHz, $CDCl_3$) δ (ppm): 1.2 (4H, m), 2.8 (1H, m), 4.5 (2H, d, J=6Hz), 7.8 (1H, broad-NH), 8.2 (1H, s).

3-Formylaminomethyl-5-methyl-1,2,4-oxadiazole.

H-NMR (60 MHz, $CDCl_3$) δ (ppm); 2.6 (3H, s), 4.6 (2H, d, J=3 Hz), 7.4 (1H, broad-NH), 8.25 (1H, s).

3-Formylaminoethyl-5-methoxymethyl-1,2,4-oxadiazole H-NMR (60 MHz, $CDCl_3$) δ (ppm): 3.5 (3H, s), 4.7 (4H, s+d, J=6 Hz), 7.8 (1H, broad-NH), 8.25 (1H, s).

c. 5-Cyclopropyl-3-isocyanomethyl-1,2,4-oxadiazole

A stirred solution of 5-cyclopropyl-3-formylamino-methyl-1,2,4-oxadiazole (60 mmol) and triethylamine (176 mmol) in $CH_2Cl_2$ (100 ml) was charged dropwise with $POCl_3$ (60 mmol) at $0^oC$. The mixture was then left for 30 minutes with stirring at $0^oC$, whereafter a solution of $Na_2CO_3$ (60 mmol) in $H_2O$ (50 ml) was added. The mixture was heated to room temperature, whereafter the organic phase was separated, dried and evaporated in vacuo. The residue was treated with ether, decanted and the solution was evaporated to give the title compound as an oil.

The oil was processed without any further purification.

IR: $cm^{-1}$: 2160.

5-Ethyl-3-isocyanomethyl-1,2,4-oxadiazole, 5-methyl-3-isocyanomethyl-1,2,4-oxadiazole, and 5-methoxymethyl-3-isocyanomethyl-1,2,4-oxadiazole were prepared in a similar manner. All compounds were oils and were characterized by their IR stretching band at 2160 $cm^{-1}$.

C. 3-(5-Ethyl-1,2,4-oxadiazole-3-yl)-5,6-dihydro-5-methyl-6-oxo--4H-7-trifluoromethyl-imidazo(1,5-a)(1,4)benzodiazepine

3,4-dihydro-4-methyl-6-trifluoromethyl-2H-1,4-benzodiazepine-2,5(1H)dione (2 mmol) was dissolved in 15 ml of dry dimethyl formamide (DMF) and charged with 2.5 mmol of K-t-butylate. The solution was cooled under $N_2$ to $20^oC$, whereafter 2.6 mmol of chlordiethylphosphate was added.

The reaction mixture was kept under $N_2$ with stirring at -20°C and charged with a -30°C cold solution of 5-ethyl-3-isocyanomethyl-1,2,4-oxadiazole (2.7 mmol) prepared as described above and K-t-butylate 2.6 mmol in 15 ml of dry DMF.

The resulting mixture was allowed to heat to room temperature, whereafter it was evaporated to dryness in vacuo. The oily residue was treated with $H_2O$/ether. The organic phase was evaporated to dryness in vacuo and the residue was crystallized from diethyl ether giving 50 mg of the title compound.

M.p. 230.4-231.3°C.

3-(3-Ethyl-1,2,4-oxadiazole-5-yl)-7-methyl-5,6-dihydro-5-methyl-6-oxo-4H-imidazo[1,5-a][1,4]benzodiazepine, M.p. 164°C, was prepared in the same manner from 6-methyl-3,4-dihydro-4-methyl-2H-1,4-benzodiazepine-2,5(1H)-dione by reaction with 3-ethyl-5-isocyanomethyl-1,2,4-oxadiazole prepared as described above.

Claims

1. Oxadiazole imidazobenzodiazepine derivatives, characterized in that they have the general formula I

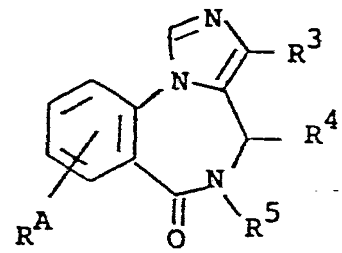

(I)

wherein $R^3$ has the formula

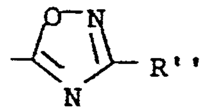

or

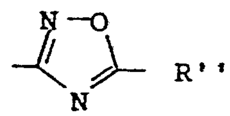

wherein

R'' is hydrogen, $C_{1-6}$-alkyl, $C_{1-6}$-alkoxymethyl, $C_{3-6}$-cycloalkyl; $R^4$ is hydrogen;

$R^5$ is $C_{1-6}$-alkyl or $R^4$ and $R^5$ together form a 2-4 membered alkylene chain; and

$R^A$ is $C_{1-6}$-alkyl, $C_{1-6}$-alkoxy or $C_{1-3}$-trifluoroalkyl.

2. An oxadiazole imidazobenzodiazepine derivative as in claim 1, characterized in that it is (s)-1-(3-cyclopropyl-1,2,4-oxadiazole-5-yl)-8-methyl-10,11,12,12a-tetrahydro-9-oxo-9H-imidazo(1,5-a)azeto(2,1-c)(1,4)benzodiazepine.

3. An oxadiazole imidazobenzodiazepine derivative as in claim 1, characterized in that it is 3-(5-ethyl-1,2,4-oxadiazole-3-yl)-5,6-dihydro-5-methyl-6-oxo-7-trifluoromethyl-4H-imidazo(1,5-a)(1,4)benzodiazepine.

4. A method of preparing a compound according to claim 1, characterized in

a) reacting a reactive derivative of a compound having the general formula (III)

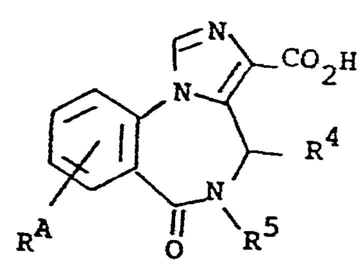

(III)

wherein $R^4$, $R^5$ and $R^A$ have the meanings defined in claim 1, with a compound having the formula (IV)

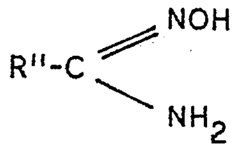

(IV)

wherein R'' has the meaning defined in claim 1, to form a compound having the formula (I) in which $R^3$ is

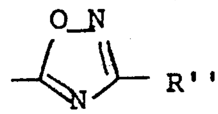

wherein R'' has the meaning defined in claim 1,

b) reacting a compound having the general formula (V)

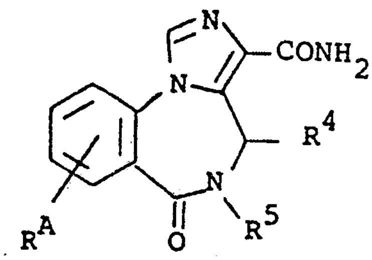

(V)

wherein $R^A$, $R^4$ and $R^5$ have the meanings defined in claim 1, with a compound of the formula VI

$$R''\text{-}C(OCH_3)_2N(CH_3)_2 \qquad (VI)$$

wherein R'' has the meaning defined in claim 1, to form a compound having the general formula (VII)

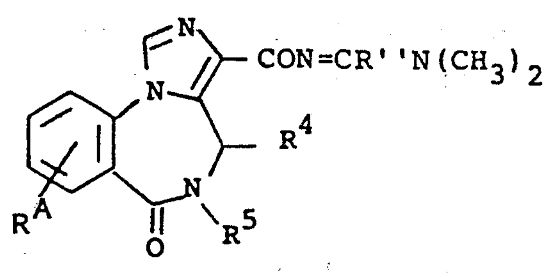

(VII)

wherein $R^A$, $R^4$, $R^5$ and R'' have the meanings defined in claim 1 and reacting the compound having the formula (VII) with $NH_2OH$ or another aminating agent, to form a

compound having the formula (I) in which $R^3$ is

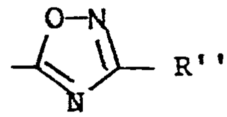

wherein R'' has the meaning defined in claim 1,

c) reacting a compound having the general formula (VIII)

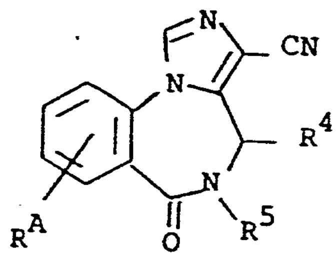

(VIII)

wherein $R^A$, $R^4$ and $R^5$ have the meanings defined in claim 1 with $NH_2OH$, to form a compound having the general formula (IX)

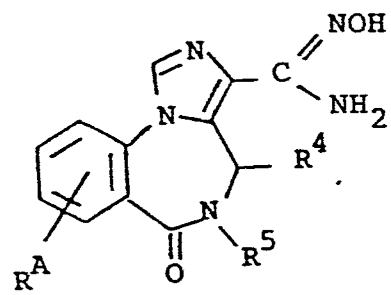

(IX)

wherein $R^A$, $R^4$ and $R^5$ have the meanings defined in claim 1, and reacting the compound having the formula (IX) with a compound having the general formula (X)

$$(R''CO)_2O \qquad (X)$$

wherein R'' has the meaning defined in claim 1, to form a compound having the formula (I) in which $R^3$ is

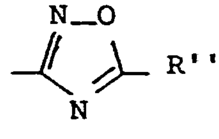

wherein R'' has the meaning defined in claim 1, or

d) reacting a compound having the general formula (XI)

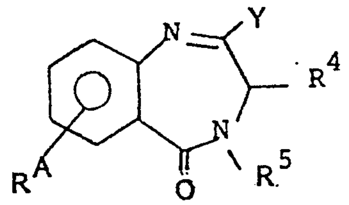

(XI)

wherein $R^4$, $R^5$ and $R^A$ have the meanings defined above, and Y is a leaving group with a compound having the formula (XII)

$$CN - CH_2 - R^3 \qquad (XII)$$

wherein $R^3$ has the meaning defined above to form a compound having the formula (I).

5. A novel compound for use in the method according to claim 4, characterized in that it has the general formula

$$CN - CH_2 - R^3$$

wherein $R^3$ has the formula

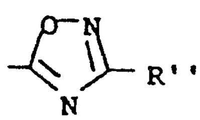

or

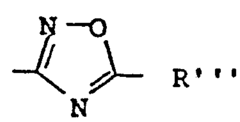

wherein R'' is hydrogen, $C_{1-6}$-alkyl, $C_{1-6}$-alkoxymethyl or $C_{3-6}$-cycloalkyl; and R''' is $C_{1-6}$-alkyl, $C_{1-6}$-alkoxymethyl, $C_{3-6}$-cycloalkyl with the proviso that R''' is not cyclopropyl.

European Patent Office

# EUROPEAN SEARCH REPORT

0197282

Application number

EP 86 10 2535

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 109 921 (SCHERING)<br>* Claim 1 *<br>--- | 1 | C 07 D 487/04<br>C 07 D 487/14<br>C 07 D 271/06 //<br>A 61 K 31/55<br>(C 07 D 487/04<br>C 07 D 243:00<br>C 07 D 235:00 )<br>(C 07 D 487/14<br>C 07 D 243:00<br>C 07 D 235:00<br>C 07 D 209:00 )<br>(C 07 D 487/14<br>C 07 D 243:00<br>C 07 D 235:00<br>C 07 D 205:00 )<br>(C 07 D 487/14<br>C 07 D 243:00<br>C 07 D 235:00<br>-/- |
| P,D X | EP-A-0 150 040 (HOFFMANN LA ROCHE)<br>* Claims 1,5 *<br>----- | 1 | |
| | | | **TECHNICAL FIELDS SEARCHED (Int. Cl.4)** |
| | | | C 07 D 487/00<br>C 07 D 271/00<br>A 61 K 31/00 |

The present search report has been drawn up for all claims

| Place of search | Date of completion of the search | Examiner |
|---|---|---|
| THE HAGUE | 30-06-1986 | ALFARO I. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

**EUROPEAN SEARCH REPORT**

0197282

Application number

EP 86 10 2535

Page 2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| | | | C 07 D 221:00 ) |
| | | | TECHNICAL FIELDS SEARCHED (Int. Cl.4) |
| | | | |

The present search report has been drawn up for all claims

| Place of search | Date of completion of the search | Examiner |
|---|---|---|
| THE HAGUE | 30-06-1986 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82